Europäisches Patentamt

**European Patent Office**

**Office européen des brevets**

(11) Numéro de publication: **0 300 897 B1**

(12) **FASCICULE DE BREVET EUROPEEN**

(45) Date de publication de fascicule du brevet: **09.09.92** (51) Int. Cl.⁵: $A61K \ 9/22$

(21) Numéro de dépôt: **88401874.8**

(22) Date de dépôt: **20.07.88**

Le dossier contient des informations techniques présentées postérieurement au dépôt de la demande et ne figurant pas dans le présent fascicule.

---

(54) **Dispositif à libération contrôlée qui fournit une délivrance continue d'un principe actif de manière contrôlée et compositions à particules multiples contenant de tels dispostifs.**

---

(30) Priorité: **21.07.87 GB 8717168**

(43) Date de publication de la demande:
**25.01.89 Bulletin 89/04**

(45) Mention de la délivrance du brevet:
**09.09.92 Bulletin 92/37**

(84) Etats contractants désignés:
**AT BE CH DE ES FR GR IT LI LU NL SE**

(56) Documents cités:
**EP-A- 0 040 457**
**EP-A- 0 169 105**
**EP-A- 0 171 457**
**GB-A- 2 189 702**

(73) Titulaire: **ROUSSEL-UCLAF**
**35, Boulevard des Invalides**
**F-75007 Paris(FR)**

(72) Inventeur: **Stead, James Arnold**
**22, Chedworth Gate Broome Manor**
**Swindon Wiltshire(GB)**
Inventeur: **Nabahi, Shohre**
**73, Manor Farm High Street**
**Watchfield Swindon Wiltshire(GB)**

(74) Mandataire: **Vieillefosse, Jean-Claude et al**
**Département des Brevets ROUSSEL UCLAF**
**B.P no 9**
**F-93230 Romainville(FR)**

## Description

La présente invention concerne un dispositif à libération contrôlée qui fournit une délivrance continue d'un principe actif à partir de celui-ci de manière contrôlée et des compositions à particules multiples contenant de tels dispositifs.

Les documents U.S. 3 854 770 et U.S. 3 916 899 décrivent tous les deux un dispositif pour libérer un agent bénéfique par lequel l'agent bénéfique est entouré par une membrane semi-perméable, à travers laquelle est prévue une voie de passage. En cours de fonctionnement, le dispositif est placé dans un fluide externe qui passe à travers ou diffuse à travers la membrane et dissout l'agent bénéfique. Un gradient de pression osmotique à travers la membrane est ainsi établi, ayant pour résultat que la solution d'agent bénéfique est délivrée à travers la voie de passage dans le fluide environnant.

Les dispositifs décrits dans les documents U.S. 3 845 770 et U.S. 3 916 899 sont destinés principalement à l'usage pharmaceutique en tant que doses unitaires, comme par exemple des comprimés revêtus d'une membrane semi-perméable. On considère en général les formes unitaires comme mal adaptées en tant qu'agents à libération prolongée, car elles font preuve d'une tendance caractéristique à se coincer à divers endroits dans le système gastro-intestinal. Dans un tel cas, la libération du principe actif peut se produire dans une zone très localisée, et le principe actif lui-même peut ne pas être acheminé jusqu'au site d'activité souhaité. En outre, la présence de concentrations aussi élevées de principe actif dans une zone définie du corps est susceptible de provoquer une irritation locale. Les formes de doses unitaires qui comptent sur le revêtement pour réaliser une libération modifiée sont aussi particulièrement vulnérables à la rupture et dans le cas d'une rupture, il y a tout lieu de croire qu'il se produit également une libération locale massive de principe actif. Un inconvénient supplémentaire des formes unitaires est leur dépendance vis-à-vis des heures de vidange de l'estomac; en général, un comprimé ou équivalent aura tendance à être retenu dans l'estomac jusqu'à ce qu'il soit balayé involontairement par les dénommées vagues de "nettoyage".

Dans les dispositifs décrits dans les documents U. S. 3 845 770 et U. S. 3 916 899, on envisage de pouvoir former la voie de passage ou le pore dans la membrane semi-perméable par perforation pendant l'assemblage du dispositif. Dans un dispositif apparenté décrit dans le document U. S. 4 278 087, on envisage la possibilité d'une perforation effectuée au moyen d'un laser. Alors qu'il est possible d'exécuter la perforation au laser aisément et avec précision sur des formes unitaires comme des comprimés, il est cependant beaucoup plus difficile et donc beaucoup plus cher en termes de temps, en pratique, d'obtenir un résultat aussi réussi avec les pellets beaucoup plus petits utilisés couramment dans les compositions à particules multiples.

Dans tous les cas, le fait lui-même que les dispositifs mentionnés ci-dessus exigent qu'un orifice soit formé, par exemple par perforation du revêtement avant l'emploi, représente en lui-même un inconvénient considérable.

Dans le document EP-A 0 171 457, on a effectué des tentatives pour surmonter cette difficulté en fournissant un moyen pour que les pores puissent être générés dans la membrane in situ. Ainsi, des granules hydrosolubles sont dispersés dans toute la membrane semi-perméable. Lorsque l'on place la composition résultante dans un environnement aqueux, les granules se dissolvent et donnent par là naissance aux pores exigés. Le document EP-A 0 169 105 décrit un dispositif analogue.

Cependant, le rapport du matériau formant des pores au matériau semi-perméable dans les documents EP-A 0 169 105 et EP-A 0 171 457 est élevé, ce qui donne naissance à une multiplicité de pores ou, dans le cas du document EP-A 0 169 105 à un réseau de canaux entrelacés discontinus liés par l'intermédiaire de passages tortueux et répartis dans toute la membrane de revêtement. Une telle prépondérance de pores dans ce revêtement suggère que la libération du principe actif de ces compositions doit être principalement à diffusion contrôlée. Les résultats cités dans les documents EP-A 0 169 105 et EP-A 0 171 457 montrent que la libération complète du principe actif à partir des compositions particulières qui y sont décrites se déroule dans un intervalle de temps relativement court. Ausi, alors qu'une libération suffisamment contrôlée du principe actif sur une période de temps prolongée pourrait être possible en principe avec les compositions fortement poreuses décrites dans les documents EP-A 0 169 105 et EP-A 0 171 457, lorsque les compositions utilisées sont de grandes formes de doses unitaires, il semble improbable que l'on puisse obtenir un tel degré de contrôle avec les pellets beaucoup plus petits qui sont habituellement présents dans les compositions à particules multiples.

Le document EP-A-0 040 457 décrit une composition comprenant un noyau central divisé en 2 compartiments, ledit noyau central étant entouré par une membrane semi-perméable constituée d'agents capables de former des pores. Il est par conséquent souhaitable de faire en sorte que la prise de fluide externe et la libération concomitante du principe actif soient régies principalement par l'osmose, car le contrôle osmotique est soumis à une cinétique d'ordre zéro, et maintient par là une libération de principe actif beaucoup plus constante, au cours d'une période de temps prolongée que ne le faire le contrôle par diffusion.

Nous avons découvert maintenant, grâce à un choix convenable de conditions selon lesquelles on effectue le revêtement, qu'il est possible d'obtenir un, ou au plus deux pores par dispositif, pour la plupart (environ 75%) des dispositifs. Cette restriction du nombre de pores garantit que la libération du principe (des principes) hydrosolubles se déroule par contrôle osmotique ; cela signifie qu'il est possible de maintenir une libération d'ordre zéro au cours d'une période de temps prolongée, et de libérer ainsi le principe souhaité à une vitesse contrôlée, constante. Ceci fait contraste avec la situation que l'on rencontre dans le document EP-A 0 169 105 dans lequel, comme on l'a mentionné ci-dessus, la membrane fortement poreuse, réticulée permet une libération totale rapide du principe actif au cours d'une échelle de temps relativement courte selon un procédé apparemment à diffusion contrôlée.

Ainsi, selon une caractéristique de la présente invention, on prévoit un dispositif à libération contrôlée constitué d'un noyau comprenant un ou plusieurs principes hydrosolubles, au moins l'un desdits principes hydrosolubles étant le principe que l'on désire libérer de manière contrôlée, ledit noyau étant entouré par une membrane de revêtement semi-perméable dans laquelle (est) sont dispersée(s) une (des) particule(s) hydrosoluble(s) d'un nombre tel qu'elle(s) donne(nt) naissance par dissolution, lorsque l'on place le dispositif dans un environnement aqueux, à un ou deux pores s'étendant à travers le revêtement et permettant la communication entre le noyau et l'extérieur du dispositif.

Le dispositif peut être, par exemple, un pellet comme par exemple un pellet formant un élément d'une composition à particules multiples.

On reconnaîtra que le pore ou les pores devrait (devraient) être suffisamment petit(s) pour garantir que le flux d'eau à travers le revêtement vers le noyau se déroule sensiblement par osmose (le gradient osmotique nécessaire provenant de la dissolution du principe (des principes) hydrosoluble(s) à l'intérieur du noyau), mais suffisamment grand pour garantir l'empêchement de la rupture prématurée du revêtement, qui résulterait d'une pression excessive créée à l'intérieur du dispositif.

A l'emploi, chaque particule hydrosoluble dans le revêtement se dissout et fournit par là un pore de libération. Pendant ce temps, l'eau pénètre dans le noyau à travers la membrane semi-perméable, à la suite de quoi les constituants du noyau se dissolvent dans l'eau et génèrent une pression osmotique à l'intérieur du noyau. Il en résulte la libération de (des) principe(s) dissous vers l'extérieur à travers le pore formé par dissolution de la particule hydrosoluble dans le revêtement.

Comme on l'a mentionné ci-dessus, les dispositifs selon l'invention peuvent se présenter sous forme de composants d'une composition à particules multiples. Ainsi, selon une caractéristique supplémentaire de la présente invention, on prévoit des compositions comprenant une pluralité de dispositifs à libération contrôlée selon l'invention.

Comme on l'a signalé auparavant, une formulation pharmaceutique à libération prolongée, lorsqu'elle se présente sous la forme d'une composition à particules multiples, procure des avantages tels que la répartition de la dose entre de nombreuses particules individuelles, l'évacuation progressive des particules avec les aliments à partir de l'estomac, ce qui amène une répartition importante des doses unitaires dans tout le système gastro-intestinal et par conséquent une réduction au minimum de la possibilité d'effets secondaires localisés comme l'irritation qui peut survenir avec certains médicaments.

On peut préparer les dispositifs selon l'invention sous forme de pellets, en recouvrant des noyaux de pellets appropriés à l'aide d'un matériau semi-perméable contenant un agent formant des pores dispersé à l'intérieur de ce matériau. La quantité d'agent formant des pores nécessaire pour donner une moyenne d'une ou deux particule(s) formant pore par pellet peut être évaluée à partir du calcul du nombre de noyaux de pellets par unité de masse à recouvrir et du nombre de particules d'agent formant des pores par unité de masse. Ces quantités peuvent à leur tout être calculées aisément à partir des données disponibles concernant la granulométrie et la masse volumique des noyaux de pellets et des particules d'agent formant des pores. Pour un nombre de pellets donné x, le nombre de particules d'agent formant des pores sera comprise entre et x et 2x dans la partie efficace de la solution de revêtement. Si l'on souhaite porter au maximum le nombre de pellets contenant une seule particule formant pore dans le revêtement, alors le nombre de particules formant pore dans la partie efficace de la solution de matériau de revêtement sera égal à x. L'expression "partie efficace" telle qu'elle est utilisée ici représente la proportion de matériel de revêtement qui contribue directement à l'épaisseur du revêtement sur les noyaux de pellets, puisqu'en pratique, on rencontre une perte de matériau de revêtement et de particules d'agent formant des pores sur les parois et régions associées de l'appareil utilisé pour appliquer le revêtement. Ainsi, pour une épaisseur donnée de matériau de revêtement, le rapport pondéral d'agent formant des pores au matériau de revêtement peut être déterminé avec précision à partir des considérations de granulométrie et de masse volumique exposées ci-dessus.

Par conséquent, la proportion en poids d'agent formant des pores au matériau de revêtement, dans le pellet fini est comprise entre 1 pour 40 et 1 pour 6000, mais elle est de préférence de l'ordre de 1 pour 300.

Il faut noter que le matériau de revêtement ne doit pas recouvrir de manière appréciable les particules d'agent formant des pores. Pour cette raison, il est préférable que le diamètre de la particule d'agent formant des pores dépasse l'épaisseur du revêtement ; néanmoins, il n'est pas nécessaire d'éliminer la condition inverse (c'est-à-dire une épaisseur de revêtement supérieure au diamètre de la particule formant pore) pourvu que, impérativement, à la suite de la dissolution de la particule hydrosoluble, le pore résultant permette un accès direct pour les constituants d'un noyau dissous vers l'extérieur du pellet.

On peut effectuer une opération de revêtement de manière commode en utilisant un tambour rotatif ou un appareil de revêtement à lit fluidisé. On peut suivre de manière commode des procédures de revêtement standard telles que celles qui sont décrites, par exemple, dans "Remington's Pharmaceutical Sciences", (les Sciences Pharmaceutiques de Remington), 16ème édition, 1980.

Selon encore une autre caractéristique de la présente invention, on prévoit un procédé de préparation de dispositifs à libération contrôlée qui comprend le revêtement de noyaux appropriés à l'aide d'un matériau semi-perméable, l'opération de revêtement étant effectuée en présence d'une quantité appropriée de particules formant des pores et pour cela, dans la majorité des dispositifs obtenus, le revêtement contient, dispersée(s) à l'intérieur de celui-ci, une (des) particule(s) hydrosoluble(s) d'un nombre tel qu'elle-(s) donne(nt) naissance par dissolution, lorsque l'on place le dispositif dans un environnement aqueux, à un ou deux pores, de préférence un pore, s'étendant à travers le revêtement et permettant la communication entre le noyau et l'extérieur du dispositif.

Selon encore une caractéristique supplémentaire de la présente invention, on prévoit une composition à particules multiples à libération contrôlée comprenant une pluralité de dispositifs, chaque dispositif ayant un noyau comprenant un ou plusieurs principes hydrosolubles, au moins l'un desdits principes hydrosolubles étant le principe que l'on souhaite libérer de manière contrôlée et chaque noyau étant entouré par une membrane de revêtement semi-perméable, dans laquelle une majorité (avantageusement au moins 75%) des dispositifs contiennent, dispersée(s) dans le revêtement semi-perméable, une (des) particule(s) hydrosoluble(s) d'un nombre tel qu'elle(s) donne(nt) naissance par dissolution, lorsque l'on place le dispositif dans un environnement aqueux, à un ou deux pores, de préférence un pore, s'étendant à travers le revêtement et permettant la communication entre le noyau et l'extérieur du dispositif.

Comme on peut le constater aisément, on peut utiliser le dispositif à libération contrôlée selon la présente invention dans une grande variété d'applications. Il sera utile partout où l'on a besoin d'un moyen par lequel on peut libérer de manière contrôlable un principe hydrosoluble dans un environnement aqueux au cours d'une période de temps prolongée. Des exemples typiques de domaines dans lesquels il est susceptible de trouver une faveur particulière incluent la médecine humaine et vétérinaire, et l'agriculture. Dans ce dernier cas, le dispositif peut être adapté de manière avantageuse pour son emploi, par exemple, dans la délivrance contrôlée de pesticides et d'engrais hydrosolubles et/ou d'oligo-éléments ou de minéraux du sol. Toutefois, les dispositifs selon l'invention sont particulièrement utiles en médecine humaine et sont décrits plus à fond en se référant particulièrement à cet usage.

Comme on l'a mentionné auparavant, les dispositifs selon l'invention peuvent prendre la forme de pellets, comme par exemple des pellets présentés sous forme de compositions à particules multiples ; l'invention sera décrit en se référant plus particulièrement aux pellets.

Parmi les matériaux de revêtement que l'on peut utiliser de manière commode, on peut citer l'acétate de cellulose, l'acétate propionate de cellulose et l'acétate butyrate de cellulose. On peut également utiliser à des fins de revêtement, d'autres polymères tels que, par exemple, le chlorure de polyvinyle, les copolymères chlorure de polyvinyl/acétate de vinyle, les poly-uréthanes, les polystyrènes, l'acétate de polyvinyle et/ou les esters d'acide polyacrylique. La membrane semi-perméable peut, le cas échéant, contenir en outre de petites quantités de plastifiant comme, par exemple, du PEG 400 ou du phtalate de dibutyle.

Parmi les solvants que l'on peut utiliser de manière commode, pour le revêtement, on peut citer, par exemple, l'acétone, la méthyléthylcétone, le chlorure de méthylène, le méthanol et des mélanges d'acétone et de méthyléthylcétone.

Parmi les matériaux que l'on peut utiliser de manière caractéristique en tant qu'agent formant des pores, on peut citer, par exemple, les cristaux de saccharose, de mannitol ou de sorbitol, ou de sels comme, par exemple, le chlorure de sodium ou le chlorure de potassium. Les particules d'agent formant des pores qui devraient être sensiblement insolubles dans le solvant utilisé pour le revêtement, ont couramment un diamètre compris entre 10 et 250 $\mu$m, de préférence entre 50 et 250 $\mu$m, et plus

particulièrement entre 100 et 150 $\mu$m.

Il faut noter que certains principes actifs seuls ne seront pas capable de générer sans aide un potentiel osmotique suffisamment élevé à l'intérieur du noyau du pellet. Pour cette raison, on envisage la possibilité d'incorporer dans le noyau tout principe hydrosoluble supplémentaire spécifiquement pour leur aptitude à augmenter le potentiel osmotique à l'intérieur du noyau. Comme substances hydrosolubles typiques que l'on peut inclure dans la formulation du noyau en tant qu'agents à action osmotique supplémentaires, on peut citer les sels (comme par exemple, NaCl, KCl et/ou LiCl) ; les polysaccharides, les alcools polyhydriques (comme, par exemple, le sorbitol et/ou le mannitol).

Le noyau du pellet est préparé de manière usuelle par compression ou par extrusion suivie d'une sphéronisation en employant des techniques standards telles qu'elles sont décrites, par exemples, dans "Remington's Pharmaceutical Sciences" (les Sciences Pharmaceutiques de Remington) 16ème édition, 1980. Pour leur utilisation avec des produits pharmaceutiques en vue d'une administration orale, le noyau mesure de préférence de 0,5 à 4 mm de diamètre, puisque les compositions formées à partir de noyaux qui dépassent 4 mm ont tendance à être retenues dans l'estomac, pouvant donner naissance localement à des effets indésirables comme on l'a mentionné ci-dessus. Dans la mesure où la libération du principe actif est un procédé contrôlé osmotiquement, le taux de libération in vivo est sensiblement indépendant de la position du pellet dans le système gastro-intestinal et il n'est pas affecté par les facteurs de l'environnement comme le pH, la mobilité intestinale et le contenu du lumen. Le taux de libération dépend uniquement de l'osmolarité et de la température de l'environnement, qui font toutes deux preuve de faibles variations physiologiques.

En vue d'une utilisation pharmaceutique, parmi les principe(s) actif(s) que l'on peut incorporer dans le noyau du pellet selon l'invention, on peut citer, par exemple, les médicaments qui affectent le système cardiovasculaire (comme par exemple les diurétiques, les béta-bloquants, les antihypertenseurs et/ou les anti-arythmiques (comme par exemple le diisopyramide), le système respiratoire (comme par exemple les bronchodilatateurs, les stéroïdes, les antitussifs, les décongestionnants systémiques et/ou les agents prophylactiques de l'asthme et d'autres troubles allergiques) et le système nerveux central, (comme par exemple les sédatifs, les anxiolytiques, les antipsychotiques, antidépresseurs (comme par exemple la trazodone), les analgésiques (comme par exemple la morphine et/ou les antiépileptiques), ainsi que les antibiotiques, les antifongiques, les hormones (comme par exemple les corticstéroïdes (comme par exemple la prednisolone et/ou les hormones sexuelles), les agents cytotoxiques, les antagonistes utilisés dans la lutte contre les maladies malignes et les médicaments (comme par exemple l'acide tiaprofénique) utilisés dans la lutte contre les maladies rhumatismales.

En outre, en vue d'une utilisation pharmaceutique, le (les) principe(s) actif(s) dans le noyau peut (peuvent) le cas échéant être présent(s) en mélange avec un ou plusieurs supports et/ou excipients pharmaceutiques. Les excipients qui facilitent la compression ou l'extrusion et la sphéronisation seront particulièrement avantageux et on peut citer, à titre d'exemple, la cellulose, le lactose, l'hydroxypropylméthylcellulose, le mannitol et le saccharose microcristallins.

De plus, en vue d'une utilisation pharmaceutique, la composition à particules multiples selon l'invention peut se trouver sous toutes formes appropriées utilisées traditionnellement dans la technique pharmaceutique, mais de préférence sous forme d'une gélule dure contenant une pluralité de pellets munis d'un revêtement selon la manière décrite ci-dessus.

Le passage suivant décrit des expériences illustratives qui ont été effectuées dans le but d'abord de démontrer des procédés typiques de préparation du noyau et ensuite d'étudier la vitesse de libération de substances à partir de compositions typiques placées dans divers milieux représentatifs. Dans les dessins annexés :

Les figures 1 et 4-8 sont des représentations graphiques montrant la quantité de substance libérée en mg en fonction du temps en heures, pour les compositions des expériences 1 et 4-8 respectivement.

Les figures 2 et 9 sont des représentations graphiques montrant le pourcentage de substance libérée en fonction du temps en heures, pour les compositions des expériences 2 et 9 respectivement , et

La figure 3 est une représentation graphique traçant la vitesse de libération de substance en mg/h en fonction du temps en heures, pour la composition de l'expérience 3.

On utilise ici les abréviations suivantes :

HPMC = hydroxypropylméthylcellulose
CA = acétate de cellulose
CAP = acétate propionate de cellulose
CAB = acétate butyrate de cellulose
DBP = phtalate de dibutyle
PEG = polyéthylène glycol.

La quantité d'acétate de cellulose à laquelle on fait référence ci-dessous en tant que CA-398-3 peut être obtenue auprès de Eastman Kodak. Les diverses qualités d'acétate propionate de cellulose, désignées ci-dessous par Cellit PR-500, PR-600 et PR-800 et d'acétate butyrate de cellulose (désignée ci-dessous en tant que CAB-500-5) peuvent être obtenues auprès de Bayer.

## 1. PREPARATION DU NOYAU.

### A. Noyaux préparés par compression.

On a préparé des mélanges de poudres en utilisant des composés à action osmotique et des colorants hydrosolubles (pour représenter le médicament) et on les a comprimé en utilisant un équipement de 3,2 mm.

Exemples :

(a) Chlorure de potassium et carmosine
(b) Chlorure de lithium et tartrazine
(c) Chlorure de potassium et bleu de méthylène

### B. Noyaux préparés par extrusion.

On a préparé des mélanges de poudres en utilisant des composés à action osmotique, d'autres excipients et des colorants hydrosolubles (pour représenter le médicament) par granulation et extrusion. L'extrudat a ensuite été sphéronisé et on a séparé les pellets de diamètres différents (fractionnement par taille).

Exemples :

(a) Chlorure de potassium, Avicel, lactose, HPMC, colorant carmosine
(b) Mannitol, Avicel RC 591, HPMC, carmosine

### 2. EXEMPLES DE REVETEMENT.

On a placé un échantillon de 2,0 g du noyau (granulométrie de 1-2 ou 2-3 mm) dans un appareil à revêtir de petite capacité et on l'a revêtu à l'aide d'un pistolet de vaporisation. On a mesuré la vitesse de libération de la carmosine en plaçant des échantillons de 0,5 g du produit dans des milieux de dissolution à diverses valeurs de pH et à diverses pressions osmotiques.

Expérience 1 :
On a revêtu environ 1,5 g de pellets de chlorure de potassium (2-3 mm) à l'aide de 25 ml de solution de revêtement ayant la formulation suivante :

| Matériau | Quantité |
|---|---|
| CA (CA-398-3) | 5 g |
| CAP (Cellit PR-500) | 5 g |
| DBP | 2,5 g |
| Acétone jusqu'à | 200 ml |

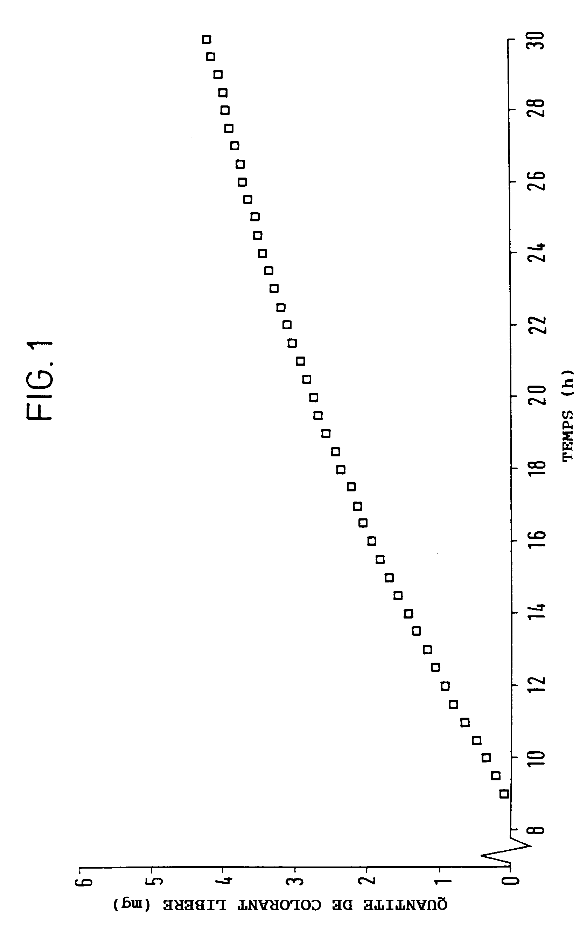

L'agent formant des pores est constitué par 5 mg de sucre en poudre (granulométrie de 125-180 microns ($\mu$m). Les pellets se revêtent bien en donnant un revêtement lisse et solide. Ensuite, on mesure le poids des pellets et constate que le poids des pellets a augmenté de 70,4 mg. On place ensuite les pellets dans un milieu de chlorure de sodium aqueux à 0,9%. Comme on peut le voir d'après la figure 1, 50% du colorant a été libéré en l'espace de 20 heures d'une vitesse d'ordre zéro après un temps de latence de 9 heures.

Expérience 2 :

On a revêtu des pellets de chlorure de potassium (granulométrie de 2-3 mm) à l'aide de 15 ml de solution de revêtement ayant la formulation suivante :

| Matériau | Quantité |
|---|---|
| CA (CA-398-3) | 1 g |
| CAP (Cellit PR-500) | 1 g |
| DBP | 0,5 g |
| Acétone jusqu'à | 40 ml |

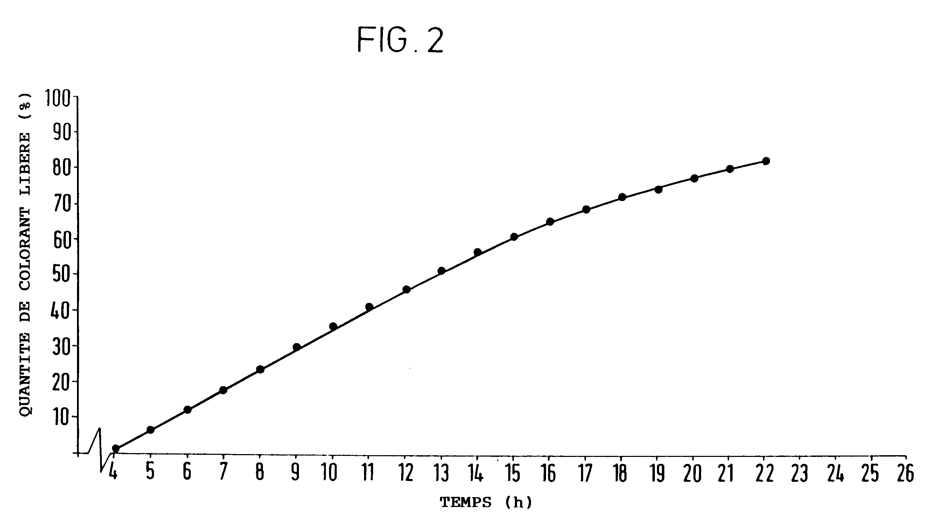

L'agent formant des pores est constitué par du sucre en poudre (granulométrie de 125-180 microns ($\mu$m). Les pellets sont bien revêtus en donnant un aspect lisse et brillant. On place ensuite les pellets dans un milieu d'eau disitllée. Comme on peut le voir d'après la figure 2, 80% du colorant a été libéré en l'espace d'environ 18 heures d'une vitesse d'ordre zéro après un temps de latence de 4 heures.

Expérience 3 :

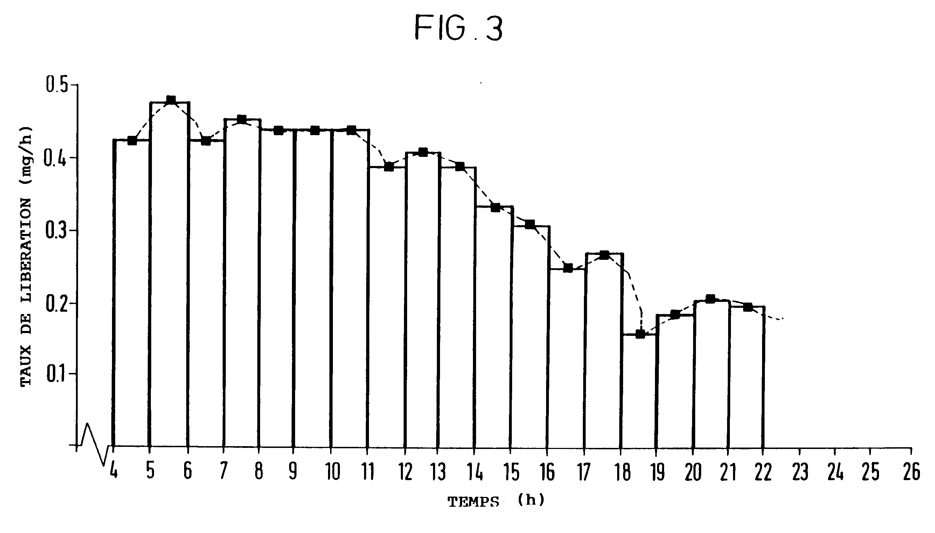

La figure 3 montre la quantité de colorant libérée par heure à partir de la composition de l'expérience 2.

Expérience 4 :

On a comparé les vitesses de dissolution de pellets revêtus à l'aide de différentes quantités de la même solution de revêtement.

| Matériau | Quantité |
|---|---|
| CAP (Cellit PR-500) | 11 g |
| DBP | 2,75 g |
| Acétone jusqu'à | 220 ml |

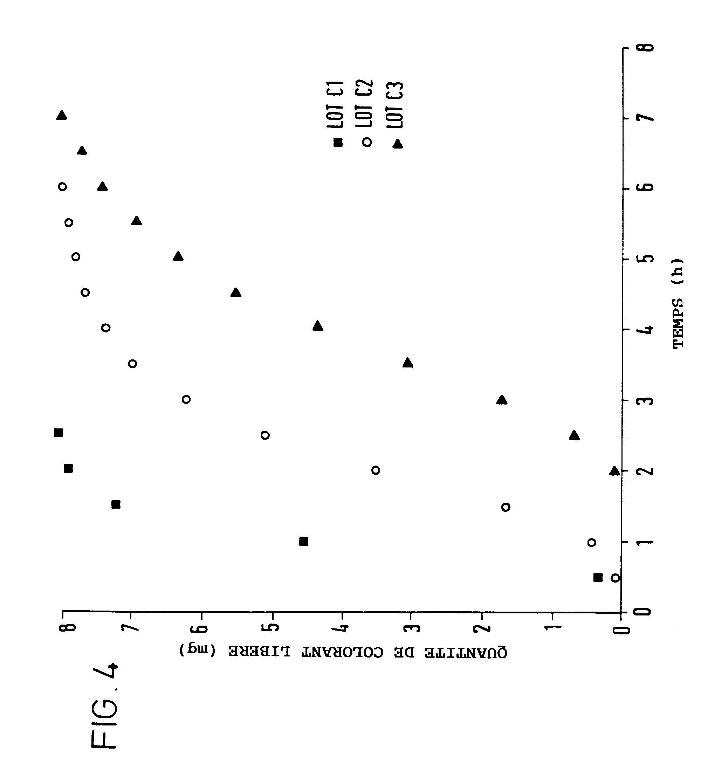

On s'est servi d'environ 2 g de pellets de chlorure de potassium (granulométrie de 2-3 mm) pour chaque revêtement. Le lot C1 (désigné par ■ à la figure 4) est revêtu à l'aide de 15 ml de solution de revêtement, le lot C2 (désigné par 0 à la figure 4) à l'aide de 20 ml et le lot C3 (désigne par ▶ à la figure 4) à l'aide de 25 ml. Dans tous les cas, l'agent formant des pores est constitué de 5 mg de sucre en poudre (granulométrie de 125-180 microns ($\mu$m). Les pellets, à la suite du revêtement présentent un bel aspect, bien qu'un meilleur contrôle des conditions de revêtement soit nécessaire. On mesure l'épaisseur du revêtement pour chaque lot. Pour le lot C1, le revêtement a 20-40 microns ($\mu$m) d'épaisseur, pour le lot C2, 40-60 um et pour le lot C3, 60-80 micron ($\mu$m). On place ensuite les pellets dans un milieu d'eau distillée. Comme on peut le voir d'après la figure 4, une augmentation de l'épaisseur de revêtement provoque un allongement du temps de libération.

Expérience 5 :

On a comparé les vitesses de dissolution de pellets revêtus avec différentes quantités de la même solution de revêtement.

| Matériau | Quantité |
|---|---|
| CA (CA-398-3) | 5,25 g |
| CAP (Cellit PR-500) | 5,25 g |
| PEG 400 | 2,63 g |
| Acétone jusqu'à | 210 ml |

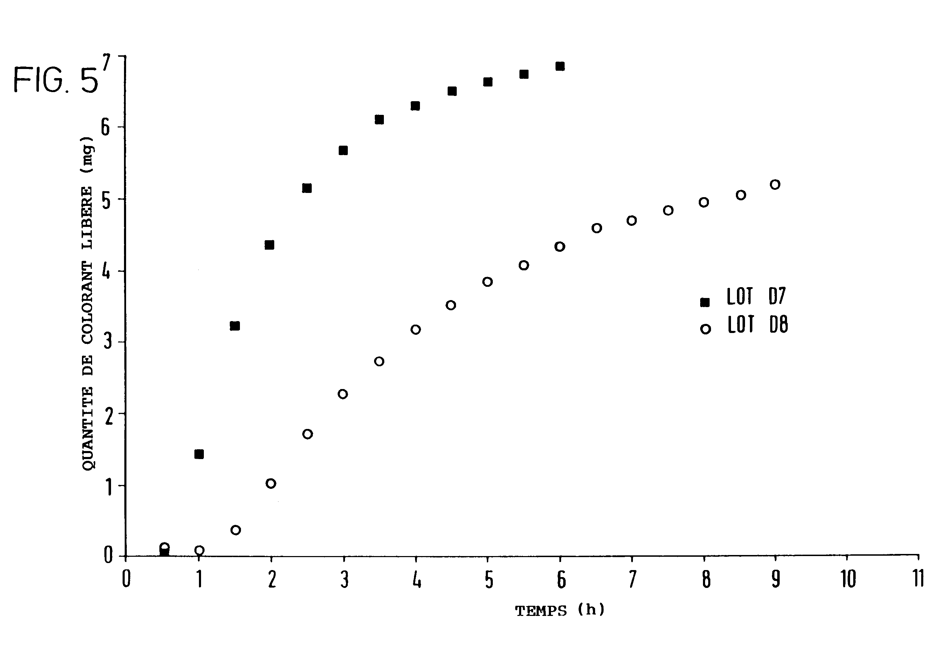

On s'est servi d'environ 2 g de pellets de chlorure de potassium (granulométrie de 2-3 mm) pour chaque revêtement. Le lot D7 (désigné par ■ à la figure 5) est revêtu à l'aide de 20 ml de solution de revêtement, le lot D8 (désigné par 0 à la figure 5) à l'aide de 25 ml de solution de revêtement. Dans les deux cas, l'agent formant des pores utilisé est constitué de 5 mg de sucre en poudre (granulométrie de

125-180 micron (μm). Les pellets, à la suite du revêtement, présentent un très bel aspect avec un revêtement solide, lisse et brillant. On pèse ensuite les pellets revêtus. Le poids de revêtement est de 17,2 mg et de 28,2 mg pour les lots respectivement D7 et D8. On place ensuite les pellets dans un milieu d'HCl 0,1N isotonique. Comme on peut le voir d'après la figure 5, l'augmentation de l'épaisseur de revêtement provoque un allongement du temps de libération.

Expérience 6 :

L'expérience 6 est analogue à l'expérience 5, les seules différences étant la formulation de la solution de revêtement et la quantité de revêtement.

| Matériau | Quantité |
|---|---|
| CA (CA-398-3) | 5 g |
| CAP (Cellit PR-600) | 5 g |
| DBP | 2,5 g |
| Acétone jusqu'à | 200 ml |

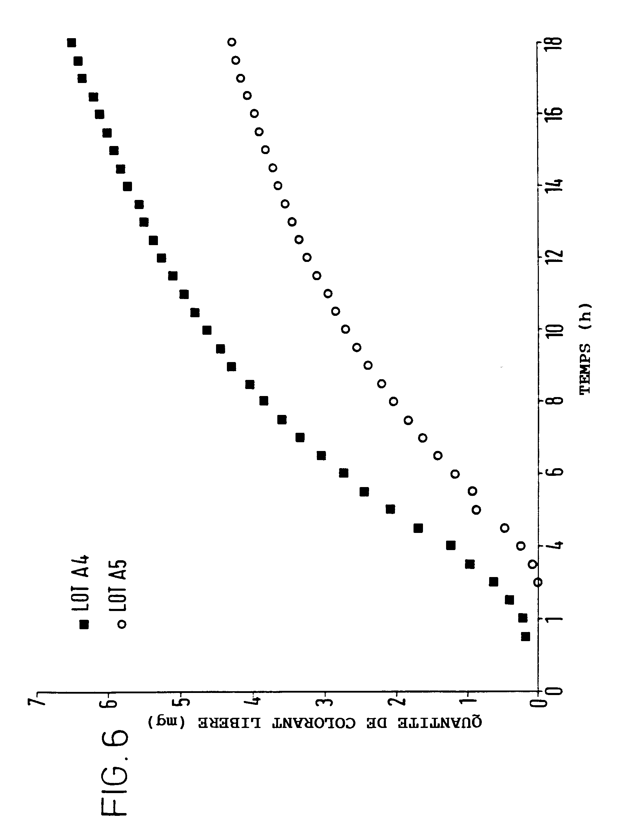

Le lot A4 (désigné par ■ à la figure 6) a été revêtu à l'aide de 15 ml de solution de revêtement et le lot A5 (désigné par 0 à la figure 6) à l'aide de 20 ml de solution de revêtement. Dans les deux cas, l'agent formant des pores utilisé est constitué de 5 mg de sucre en poudre (granulométrie de 90-125 microns (μm). On pèse les pellets à la suite du revêtement et on constate que leurs poids a augmenté respectivement de 27,4 mg et de 46 mg. On a mesuré l'épaisseur du revêtement en utilisant un microscope et on a obtenu des valeurs respectivement de 10-20 microns (μm) et de 20-30 microns (μm). On place ensuite les pellets dans un milieu d'HCl 0,1N isotonique. Comme on peut le voir d'après la figure 6, l'augmentation de l'épaisseur du revêtement provoque un allongement du temps de libération.

Expérience 7 :

On a comparé les vitesses de dissolution de pellets revêtus avec différentes quantités de solution de revêtement.

| Matériau | Quantité |
|---|---|
| CA (CA-398-3) | 5,25 g |
| CAP (Cellit PR-500) | 5,25 g |
| PEG 400 | 2,63 g |
| Acétone jusqu'à | 210 ml |

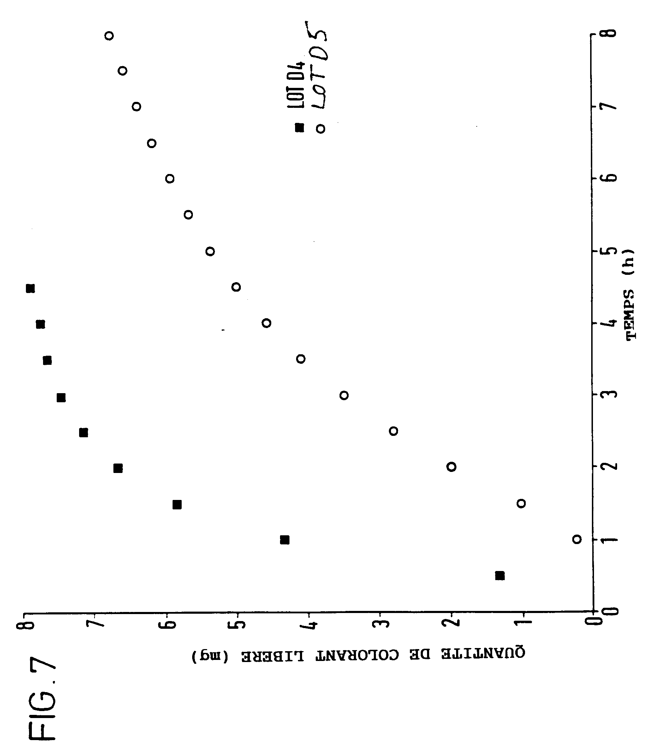

On s'est servi d'environ 2 g de pellets de chlorure de potassium pour chaque revêtement. Le lot D4 (désigné par ■ à la figure 7) est revêtu à l'aide de 15 ml de solution de revêtement, le lot D5 (désigné par 0 à la figure 7) à l'aide de 20 ml de solution de revêtement. Dans les deux cas, l'agent formant des pores utilisé est constitué de 5 mg de sucre en poudre (granulométrie de 90-125 microns (μm). Les épaisseurs des revêtements sont respectivement de 20-40 microns (μm) et de 60-80 microns (μm) pour les lots D4 et D5. On place ensuite les pellets dans un milieu de chlorure de sodium aqueux à 0,9%. Comme on peut le voir d'après la figure 7, l'augmentation de l'épaisseur de revêtement provoque un allongement du temps de libération.

Expérience 8 :

On a comparé les vitesses de dissolution d'un lot de pellets dans deux différents milieux.

| Matériau | Quantité |
|---|---|
| CA (CA-398-3) | 11 g |
| PEG 400 | 2,75 |
| DBP | 2,75 g |
| Acétone jusqu'à | 220 ml |

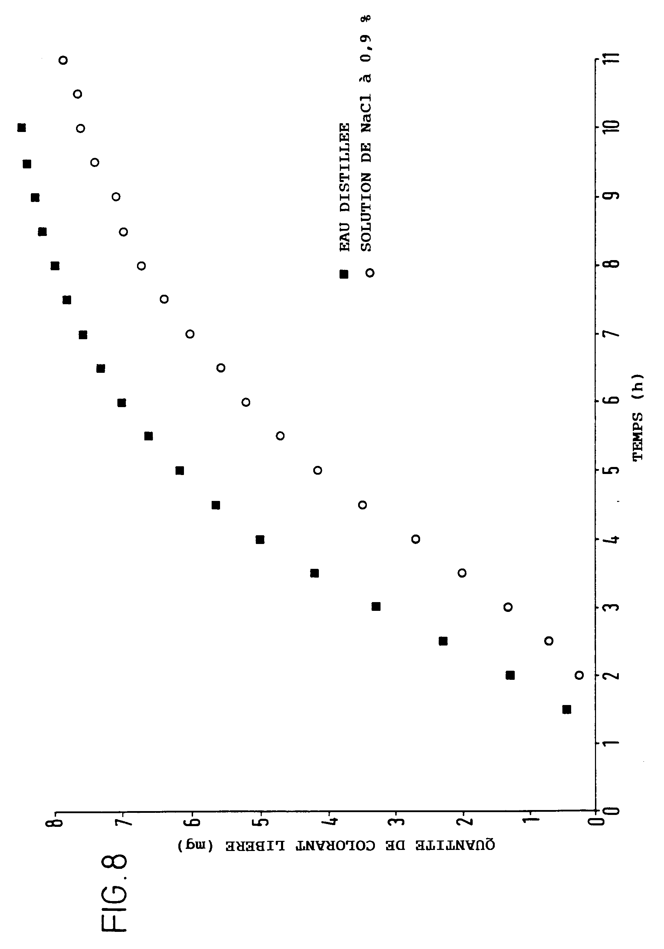

On a revêtu 2 g de pellets de chlorure de potassium à l'aide de 25 ml de solution de revêtement ci-dessus, l'agent formant des pores utilisé est constitué de 5 mg de sucre en poudre (granulométrie de 125-180 microns ($\mu$m). On effectue l'essai de dissolution dans de l'eau distillée (désigné par ■ à la figure 8) et dans une solution de chlorure de sodium à 0,9% (désigné par 0 à la figure 8). Comme on peut le voir d'après la figure 8, on constate une diminution de la vitesse de libération lorsque la pression osmotique augmente.

Expérience 9 :

On a revêtu 2 g de pellets de chlorure de potassium à l'aide de 25 ml de solution de revêtement ayant la formulation suivante :

| Matériau | Quantité |
|---|---|
| CA (CA-398-3) | 2,5 g |
| CAB (CAB-500-5) | 2,5 g |
| DBP | 1,25 g |
| Acétone jusqu'à | 100 ml |

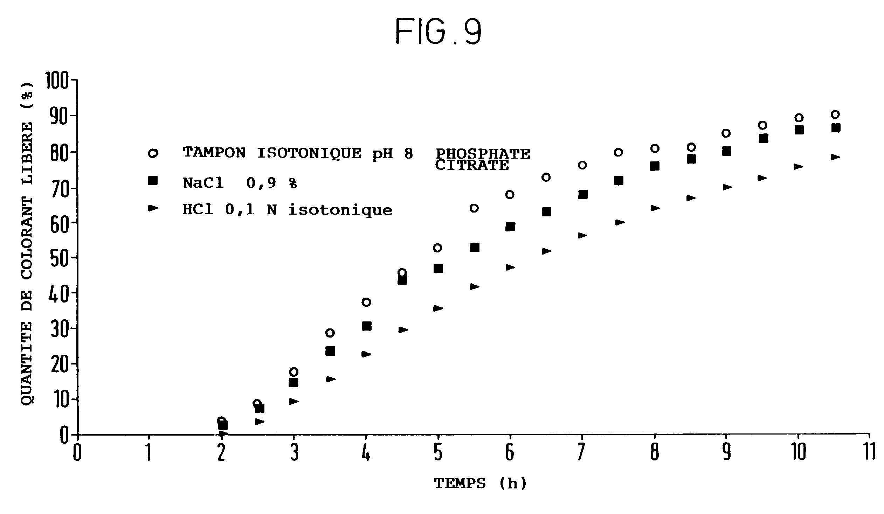

On a étudié la vitesse de libération dans des milieux à diverses valeurs de pH, les milieux choisis étant constitués par du chlorure de sodium aqueux à 0,9% (désigné par ■ à la figure 9), du tampon pH 8 citrate-phosphate isotonique (désigne par 0 à la figure 9) et de l'HCl 0,1N isotonique (désigné par ► à la figure 9). Comme on peut le voir d'après la figure 9, la vitesse de libération est sensiblement indépendante du pH pour des valeurs de pH comprises entre 1 et 8

Les exemples non limitatifs suivants servent à illustrer plus complètement la présente invention.

**Exemple 1 : Compositions contenant du chlorhydrate de trazodone.**

On a préparé des pellets de chlorhydrate de trazodone en utilisant du chlorure de sodium, de l'Avicel PH-101, du lactose 0,07 mm (200 mesh) et de la HPMC en tant qu'excipients. On utilise des pellets de 2-3 mm de diamètre pour le revêtement. On place 2,0 g de pellets dans l'appareil à revêtir, met en suspension 5 mg de particules de sucre de 125-180 microns ($\mu$m) dans 15 ml de solution de revêtement (CA-398-3, Cellit PR-500, DBP) et on revêt les pellets à l'aide de ce mélange. On effectue l'essai de dissolution sur 600 mg de pellets (équivalent à 28,0 mg de chlorhydrate de trazodone) dans un tampon phosphate-citrate de pH 6,0. Comme on peut le voir d'après les résultats suivants, environ 80% du principe actif est libéré à une vitesse d'ordre zéro en l'espace de 4 heures.

| Temps (h) | Quantité de principe actif libéré (%) |
|-----------|----------------------------------------|
| 0,5 | 1,1 |
| 1,0 | 5,1 |
| 1,5 | 15,5 |
| 2,0 | 32,5 |
| 2,5 | 50,5 |
| 3,0 | 66,1 |
| 3,5 | 77,3 |
| 4,0 | 84,1 |
| 4,5 | 89,5 |
| 5,0 | 93,5 |
| 5,5 | 95,6 |
| 6,0 | 97,5 |
| 6,5 | 98,6 |
| 7,0 | 99,6 |

**Exemple 2 : Compositions contenant du sulfate de morphine.**

On a préparé des pellets de sulfate de morphine en utilisant du chlorure de sodium, de l'Avicel PH-101, du lactose 0,07 mm (200 mesh) et de la HPMC en tant qu'excipients. On s'est servi de pellets de 1,4-2,0 mm de diamètre pour le revêtement. On place 2,0 g de pellets dans l'appareil à revêtir, met en suspension 5 mg de particules de sucre de 125-180 microns ($\mu$m) dans 25 ml de solution de revêtement (CA-398-3, Cellit PR-500, DBP) et on revêt les pellets à l'aide de ce mélange. On effectue l'essai de dissolution sur 800 mg de pellets (équivalent à 16,7 mg de sulfate de morphine) dans de l'HCl 0,001N. Comme on peut le voir d'après les résultats suivants, environ 10 mg (60%) du principe actif est libéré à une vitesse d'ordre zéro en l'espace de 20 heures.

| Temps (h) | Quantité de principe actif libéré (mg) |
|-----------|----------------------------------------|
| 0,5 | - |
| 2,0 | 1,350 |
| 4,0 | 2,925 |
| 6,0 | 4,500 |
| 8,0 | 5,625 |
| 10,0 | 6,525 |
| 12,0 | 7,200 |
| 14,0 | 7,650 |
| 16,0 | 8,325 |
| 18,0 | 9,450 |
| 19,0 | 9,900 |

**Exemple 3 : Compositionscontenant de la prednisolone.**

On a préparé des pellets de predniolone en utilisant du chlorure de sodium, de l'Avicel PH-101, du lactose 0,07 mm (200 mesh) et de la HPMC en tant qu'excipients. On utilise des pellets de 2-3 mm de diamètre pour le revêtement. On place 2,0 g de pellets dans l'appareil à revêtir et on les revêt à l'aide de 25 ml de solution de revêtement (la formulation du revêtement étant exactement la même que pour les pellets de sulfte de morphine de l'exemple 2). On effectue un essai de dissolution sur 600 mg de pellets (équivalent à 11,8 mg de prednisolone). Comme on peut le voir d'après les résultats suivants, environ 66% du principe actif est libéré à une vitesse d'ordre zéro en l'espace de 40 heures dans de l'HCl 0,1N.

| Temps (h) | Quantité de principe actif libéré (mg) |
|---|---|
| 0 | - |
| 4,0 | 0,52 |
| 8,0 | 1,35 |
| 12,0 | 2,22 |
| 16,0 | 3,08 |
| 20,0 | 3,92 |
| 24,0 | 4,72 |
| 28,0 | 5,53 |
| 32,0 | 6,33 |
| 36,0 | 7,08 |
| 40,0 | 7,83 |

## Exemple 4 : Compositions contenant de l'acide tiaprofénique.

On a préparé des pellets d'acide tiaprofénique en utilisant les mêmes excipients que ceux que l'on a utilisés pour les pellets de prednisolone de l'exemple 3. Une unité de 600 mg contient 268 mg d'acide tiaprofénique. On s'est servi de pellets de 2-3 mm de diamètre pour le revêtement. On revêt 2,0 g de pellets à l'aide de 25 ml de solution de revêtement (la formulation et la taille des particules de sucre étant les mêmes que dans les exemples précédents). On effectue l'essai de dissolution sur 600 mg de pellets dans 900 ml de tampon citrate (pH 5,50). Comme on peut le voir d'après les résultats suivants, la composition montre une libération de 40 heures à une vitesse d'ordre zéro dans le milieu ci-dessus.

| Temps (h) | Quantité de principe actif libéré (%) |
|---|---|
| 0 | - |
| 4,0 | 25,53 |
| 12,0 | 68,86 |
| 16,0 | 86,44 |
| 20,0 | 103,81 |
| 24,0 | 120,14 |
| 28,0 | 134,58 |
| 32,0 | 148,19 |
| 36,0 | 160,54 |
| 40,0 | 172,05 |

## Exemple 5 : Compositions contenant du phosphate de disopyramide.

On a préparé des pellets de phosphate de disopyramide en utilisant les mêmes excipients que ceux que l'on a utilisés pour les pellets de prednisolone de l'exemple 3. Une unité de 470 mg contient 250 mg de phosphate de disopyramide (193 mg de base). On s'est servi de pellets de 2-3 mm de met diamètre pour le revêtement. On revêt 2,0 g de pellets à l'aide de 25 ml de solution de revêtement (la formulation et la granulométrie des particules étant les mêmes que dans les exemples précédents)..On effectue l'essai de dissolution sur 400 mg de pellets dans 900 ml de HCl 0,001N. Comme on peut le voir d'après les résultats suivants, 70% du principe actif est libéré à une vitesse d'ordre zéro en l'espace de 12 heures.

| Temps (h) | Quantité de principe actif libéré (%) |
|---|---|
| 0 | - |
| 2,0 | 6,1 |
| 4,0 | 24,4 |
| 6,0 | 46,2 |
| 8,0 | 59,4 |
| 10,0 | 68,5 |
| 12,0 | 75,2 |
| 14,0 | 80,7 |
| 16,0 | 84,9 |
| 18,0 | 87,8 |
| 20,0 | 90,8 |

**Revendications**

1. Un dispositif à libération contrôlée sous forme de pellet constitué d'un noyau comprenant un ou plusieurs principes hydrosolubles, au moins l'un desdits principes hydrosolubles étant le principe que l'on souhaite libérer de manière contrôlée, ledit noyau étant entouré par une membrane de revêtement semi-perméable dans laquelle est (sont) dispersée(s) une (ou deux) particule(s) hydrosoluble(s) qui donne(nt) naissance par dissolution, lorsque l'on place le dispositif dans un environnement aqueux, à un ou deux pores s'étendant à travers le revêtement et permettant la communication entre le noyau et l'extérieur du dispositif, et dans lequel la proportion en poids d'agent formant des pores au matériau de revêtement du pellet est comprise entre 1 pour 40 et 1 pour 6000.

2. Un dispositif selon la revendication 1, dans lequel la proportion en poids d'agent formant des pores au matériau de revêtement du pellet est de l'ordre 1 pour 300.

3. Un dispositif selon l'une quelconque des revendications précédentes, dans lequel le (les) diamètre(s) de la particule (des particules) d'agent formant des pores dépasse(nt) l'épaisseur du revêtement semi-perméable.

4. Un procédé de préparation de dispositifs à libération contrôlée qui comprend le revêtement de noyaux de pellets appropriés à l'aide d'un matériau semi-perméable, l'opération de revêtement étant effectuée en présence d'une à deux particules formant pore par pellet, dans lequel, pour la majorité des dispositifs obtenus, le revêtement contient dispersée(s) à l'intérieur de celui-ci une (ou deux) particule-(s) hydrosoluble(s) qui donne(nt) naissance par dissolution, lorsque l'on place le dispositif dans un environnement aqueux à un ou deux pores s'étendant à travers le revêtement et permettant la communication entre le noyau et l'extérieur du dispositif.

5. Une composition à particules multiples à libération contrôlée comprenant une pluralité de dispositifs, chaque dispositif ayant un noyau comprenant un ou plusieurs principes hydrosolubles, au moins l'un desdits principes hydrosolubles étant le principe que l'on souhaite libérer de manière contrôlée, et chaque noyau étant entouré par une membrane de revêtement semi-perméable, dans laquelle une majorité des dispositifs contiennent, dispersée(s) dans le revêtement semi-perméable, une (ou deux) particule(s) hydrosoluble(s) qui donne(nt) naissance par dissolution, lorsque l'on place le dispositif dans un environnement aqueux, à un ou deux pores s'étendant à travers le revêtement et permettant la communication entre le noyau et l'extérieur du dispositif.

6. Une composition selon la revendication 5, dans laquelle au moins 75 % de dispositifs contiennent dispersées dans le revêtement semi-perméable une ou deux particules hydrosolubles qui donnent naissance à un ou deux pores s'étendant à travers le revêtement.

**Claims**

1. A controlled-release device in the form of a pellet constituted by a core containing one or more hydrosoluble ingredients, at least one of said hydrosoluble ingredients being the ingredient which it is

12

desired to release in a controlled manner, said core being surrounded by a semi-permeable coating membrane in which one (or two) hydrosoluble particle(s) is (are) dispersed which produces (produce) by dissolution, when the device is placed in an aqueous environment, one or two pores extending through the coating and allowing communication between the core and the outside of the device, and in which the proportion by weight of pore-forming agent in the coating material of the pellet is comprised between 1 to 40 and 1 to 6000.

2. A device according to claim 1, in which the proportion by weight of pore-forming agent in the coating material of the pellet is of the order of 1 to 300.

3. A device according to any one of the previous claims, in which the diameter(s) of the particle(s) of pore-forming agent exceeds (exceed) the thickness of the semi-permeable coating.

4. A preparation process for controlled-release devices which comprises the coating of suitable pellet cores using a semi-permeable material, the coating operation being carried out in the presence of one or two pore-forming particles per pellet, in which, for the majority of devices obtained, the coating contains, dispersed inside it, one (or two) hydrosoluble particle(s) which produces (produce) by dissolution, when the device is placed in an aqueous environment, one or two pores extending through the coating and allowing communication between the core and the outside of the device.

5. A controlled-release composition with multiple particles containing a plurality of devices, each device having a core containing one or more hydrosoluble ingredients, at least one of said hydrosoluble ingredients being the ingredient which it is desired to release in a controlled manner, and each core being surrounded by a semi-permeable coating membrane, in which a majority of the devices contain, dispersed in the semi-permeable coating, one (or two) hydrosoluble particle(s) which produce by dissolution, when the device is placed in an aqueous environment, one or two pores extending through the coating and allowing communication between the core and the outside of the device.

6. A composition according to claim 5, in which at least 75% of devices contain one or two hydrosoluble particles dispersed in the semi-permeable coating which produce one or two pores extending through the coating.

**Patentansprüche**

1. System für die kontrollierte Freigabe in Form von Pellets, bestehend aus einem Kern, welcher ein oder mehrere wasserlösliche Prinzipien umfaßt, wobei zumindest eines dieser wasserlöslichen Prinzipien das Prinzip ist, das man kontrolliert freizusetzen wünscht, wobei der Kern von einer semi-permeablen Überzugsmembran umgeben ist, in der ein (oder zwei) wasserlösliches (wasserlösliche) Teilchen dispergiert ist (sind), das (die) zu einer Auflösung führt (führen), wenn man das System in eine wäßrige Umgebung bringt, mit ein oder zwei Poren, die sich über den Überzug erstrecken und die Kommunikation zwischen dem Kern und der Außenseite des Systems ermöglichen, worin der Gewichtsanteil des die Poren bildenden Mittels zu dem Überzugsmaterial des Pellets zwischen 1:40 und 1:6000 liegt.

2. System gemäß Anspruch 1, worin der Gewichtsanteil des die Poren bildenden Mittels zu dem Überzugsmaterial des Pellets in der Größenordnung von 1:300 liegt.

3. System gemäß einem der vorhergehenden Ansprüche, worin der (die) Durchmesser des Teilchens (der Teilchen) des die Poren bildenden Mittels die Dicke des semi-permeablen Überzugs überschreitet (überschreiten).

4. Verfahren zur Herstellung von Systemen für die kontrollierte Freigabe, das das Überziehen geeigneter Kerne der Pellets mit Hilfe eines semi-permeablen Materials umfaßt, wobei das Überzugsverfahren in Anwesenheit von ein bis zwei porenbildenden Teilchen je Pellet durchgeführt wird, worin bei der Mehrzahl der erhaltenen Systeme der Überzug im Inneren derselben dispergiert ein (oder zwei) wasserlösliches (wasserlösliche) Teilchen enthält, das (die) zur Auflösung führt (führen), wenn man das System in eine wäßrige Umgebung bringt, wobei sich ein oder zwei Poren über den Überzug hinweg erstrecken und die Kommunikation zwischen dem Kern und der Außenseite des Systems ermöglichen.

**5.** Zusammensetzung aus zahlreichen Teilchen für die kontrollierte Freigabe, umfassend eine Vielzahl von Systemen, wobei ein jedes System einen Kern aufweist, der ein oder mehrere wasserlösliche Prinzipien umfaßt, wobei zumindest eines dieser wasserlöslichen Prinzipien das Prinzip ist, das man kontrolliert freizusetzen wünscht, und wobei ein jeder Kern von einer semi-permeablen Überzugsmembran umgeben ist, worin die Mehrzahl der Systeme dispergiert in dem semi-permeablen Überzug ein (oder zwei) wasserlösliches (wasserlösliche) Teilchen enthält, das (die) zur Auflösung führt (führen), wenn man das System in eine wäßrige Umgebung bringt, wobei sich ein oder zwei Poren über den Überzug hinweg erstrecken und die Kommunikation zwischen dem Kern und der Außenseite des Systems ermöglichen.

**6.** Zusammensetzung gemäß Anspruch 5, worin zumindest 75% der Systeme in dem semi-permeablen Überzug dispergiert ein oder zwei wasserlösliche Teilchen enthalten, die zu ein oder zwei Poren führen, welche sich über den Überzug hinweg erstrecken.

# FIG. 1

QUANTITE DE COLORANT LIBERE (mg)

TEMPS (h)

EP 0 300 897 B1

# FIG. 2

QUANTITE DE COLORANT LIBERE (%)

TEMPS (h)

EP 0 300 897 B1

# FIG. 3

EP 0 300 897 B1

FIG. 4

FIG. 5

FIG. 6

QUANTITE DE COLORANT LIBERE (mg)

■ LOT A4
○ LOT A5

TEMPS (h)

EP 0 300 897 B1

FIG.7

FIG.8

QUANTITE DE COLORANT LIBERE (mg)

TEMPS (h)

■ EAU DISTILLEE
○ SOLUTION DE NaCl à 0,9 %

EP 0 300 897 B1

# FIG.9

TAMPON ISOTONIQUE pH 8 PHOSPHATE CITRATE

○ NaCl 0,9 %

■ HCl 0,1 N isotonique

○ ■ ▲

QUANTITE DE COLORANT LIBERE (%)

TEMPS (h)